# EUROPÄISCHE PATENTANMELDUNG

(11) **EP 1 792 568 A1**
(43) Veröffentlichungstag der Anmeldung: **06.06.2007**
(21) Anmeldenummer: 05026526.3
(22) Anmeldetag: 05.12.2005
(51) Int. Cl.: A61B 5/151, A61B 5/155, A61B 5/00

(54) **Wieder verwendbare Stechhilfe und Verfahren zum Ausführen einer Stechbewegung mittels einer wieder verwendbaren Stechhilfe**

(71) Anmelder: F. HOFFMANN-LA ROCHE AG, 4070 Basel (CH); Roche Diagnostics GmbH, 68305 Mannheim (DE)
(72) Erfinder: Krämer, Uwe, 68549 Ilvesheim (DE); Hönes, Joachim, 64673 Zwingenberg (DE); Miltner, Karl, 67227 Frankenthal (DE); List, Hans, 64754 Hesseneck-Kailbach (DE); Keil, Michael, 67071 Ludwigshafen (DE)
(74) Vertreter: Huhn, Michael

(57) **Zusammenfassung**

Die Erfindung bezieht sich auf eine wieder verwendbare Stechhilfe (1), enthaltend eine Lanzette (2), einen Energiespeicher (5) zum Übertragen von in dem Energiespeicher (5) gespeicherter Energie auf die Lanzette (2) zur Ausführen einer Stechbewegung, eine Ladeeinrichtung zum Aufladen des Energiespeichers (5) und eine Steuereinheit, wobei die Steuereinheit so ausgelegt ist, dass sie die Ladeeinrichtung im Anschluss an die Stechbewegung ansteuert, so dass der Energiespeicher (5) im Anschluss an die Stechbewegung automatisch mit Energie für eine nächste Stechbewegung der Lanzette (2) aufgeladen wird.

## Beschreibung

Die vorliegende Erfindung betrifft eine wieder verwendbare Stechhilfe und ein Verfahren zum Ausführen einer Stechbewegung mittels einer wieder verwendbaren Stechhilfe, insbesondere ein Verfahren zum Erzeugen einer Hautöffnung in einem Körperteil.

Aus der Hautöffnung kann dann eine Körperflüssigkeit (Blut oder interstitielle Flüssigkeit) entnommen werden.

Eine Entnahme von Körperflüssigkeiten, insbesondere von Blut, wird vor allem mit dem Ziel einer nachfolgenden Analyse durchgeführt, um eine Diagnose von Krankheiten oder die Überwachung des Stoffwechselzustandes eines Patienten zu ermöglichen. Insbesondere von Diabetikern wird eine solche Entnahme durchgeführt, um die Blutzuckerkonzentration zu bestimmen. Um z.B. für Diagnosezwecke eine Entnahme von nur geringen Mengen Blut vorzunehmen, werden üblicherweise sterile, scharfe Lanzetten verwendet, die z. B. von Krankenhauspersonal oder dem Patienten selbst bei dem Patienten in die Fingerbeere oder auch andere Körperteile kurz eingestochen werden. Vor allem im Bereich des so genannten "Home-Monitoring", wobei medizinische Laien selbst einfache Analysen des Blutes durchführen, werden Lanzetten und dazu passende Geräte (so genannte Blutentnahmegeräte, Blutlanzettenvorrichtungen oder - wie sie im Folgenden genannt werden sollen - Stechhilfen), angeboten, die eine möglichst schmerzarme und reproduzierbare Blutgewinnung ermöglichen.

Im Stand der Technik sind bereits seit einiger Zeit Stechhilfen bekannt, die mechanische oder elektrische Antriebseinheiten für eine Lanzette oder eine Nadel beinhalten, mit denen der Patient oder Krankenhauspersonal eine Entnahme auf einfache Weise durchführen kann. Ein hierfür geeignetes Gerät ist beispielsweise das im Handel befindliche "Softclix", dessen Funktionsweise in der US 5,318,584 beschrieben ist. Dieses Gerät sieht eine Einstellmöglichkeit für die Einstechtiefe einer Lanzette in das Gewebe vor. Der Patient kann so die minimale Einstechtiefe wählen, mit der er eine für eine nachfolgende Analyse gerade noch ausreichende Blutmenge erhält, und dadurch den Einstichschmerz sehr niedrig halten. Bei dieser Stechhilfe überträgt eine komprimierte Feder bei einem Einstechvorgang Energie auf eine Lanzette zum Ausführen einer Stechbewegung.

US 2004/0092996 A1 bezieht sich auf ein weiteres Blutentnahmesystem mit einer durch eine Feder angetriebenen Lanzette. Diese Stechhilfe weist Mittel zum Spannen der Feder auf. Zum Antreiben der Lanzette wird durch die Feder ein Antriebsrotor angetrieben und die resultierende Drehbewegung des Antriebsrotors durch einen Kopplungsmechanismus in die Einstichbewegung der Lanzette umgesetzt.

Bei weiteren im Stand der Technik bekannten Stechhilfen wird dem Benutzer der zum Teil umständliche Vorgang des Spannens der Lanzetten, sowie das anschließende Auslösen des Stechvorgangs weitestgehend erspart. Der Patient kann über einen Knopfdruck einen elektrischen Antriebsmechanismus aktivieren, ohne dass darüber hinaus weitere Handhabungsschritte notwendig sind oder noch ein Kraftaufwand durch den Benutzer geleistet werden muss. Das Dokument WO 02/100460 offenbart ein Blutentnahmesystem, bei dem eine elektrische Antriebseinheit zum Bewegen der Lanzette dient. Die Bewegung der Lanzette wird dabei durch Steuereinheiten gesteuert, so dass eine definierte Stechbewegung erfolgen kann.

Die durch die Erzeugung einer Hautöffnung mittels einer Stechhilfe gewonnene flüssige Probe wird z.B. durch ein Analysesystem analysiert, das den Blutzuckergehalt bestimmt. Im Stand der Technik sind Analysesysteme bekannt, in denen eine Blutprobe auf einem analytischen Testelement (z.B. einem Testelement, wie es in CA 2,311,496 beschrieben ist) elektrochemisch oder photometrisch analysiert wird. Die zahlreichen, für die eigenhändige Blutzuckerbestimmung benötigten Systemkomponenten (Lanzette, Stechhilfe, Testelement und Analysegerät) benötigen jedoch viel Platz und bedingen eine relativ komplexe Handhabung. Inzwischen gibt es auch Systeme mit einem höheren Grad der Integration und damit einer einfacheren Handhabung, bei denen z.B. die Testelemente im Analysesystem magaziniert und für die Messung zur Verfügung gestellt werden. Ein integriertes Analysesystem kann ferner eine Stechhilfe beinhalten, so dass idealerweise das Erzeugen einer Hautöffnung, die Aufnahme einer Blutprobe auf ein Testelement und die Durchführung von Messungen zur Analyse der Probe auf dem Testelement automatisch durch das Analysesystem erfolgt.

WO 02/00101 offenbart ein Analysesystem mit einer Vielzahl von Nadeln, die einzeln durch eine elektrisch oder mittels einer Feder angetriebene Druckeinrichtung aus einem Gehäuse herausbewegt werden können, um eine Perforation in Haut zu erzeugen. Die so gewonnene Probe wird in dem Analysesystem analysiert.

Bei einer wieder verwendbaren, separat vorliegenden oder in ein Analysesystem integrierten Stechhilfe muss vor der Erzeugung einer Stechbewegung der Lanzette ein Energiespeicher aufgeladen werden, so dass die darin gespeicherte Energie zumindest teilweise in kinetische Energie einer Lanzette umgewandelt werden kann. Eine in dem Analysesystem als Energiespeicher vorgesehene Feder muss beispielsweise zur Erzeugung einer Stechbewegung einer Lanzette gespannt werden. Das manuelle Spannen einer Feder vor der Verwendung der Stechhilfe durch einen Anwender stellt einen zusätzlichen, zu vermeidenden Arbeitsschritt für den Anwender dar. Ein automatisches Aufladen des Energiespeichers (z.B. Spannen der Feder) in der Stechhilfe bzw. dem Analysesystem bevor die Stechhilfe aktiviert wird, bedingt entweder eine lange Wartezeit oder erfordert eine hohe Leistung z.B. eines elektrischen Antriebs und damit einen hohen Energieverbrauch, wodurch eine schnellere Entladung von vorhandenen Batterien erfolgt.

Die Aufgabe der vorliegenden Erfindung ist es daher, die Nachteile des Standes der Technik zu vermeiden und insbesondere eine Stechhilfe und ein Verfahren zum Ausführen einer Stechbewegung mittels einer wieder verwendbaren Stechhilfe bereitzustellen, bei der/dem möglichst wenig Energie pro Messzyklus erforderlich ist und insbesondere eine Stechhilfe und ein Verfahren bei der/dem die wieder verwendbare Stechhilfe zum Ausführen einer Stechbewegung, z.B. zum Erzeugen der Körperöffnung, möglichst schnell einsatzfähig ist.

Diese Aufgabe wird erfindungsgemäß gelöst durch eine wieder verwendbare Stechhilfe, enthaltend eine Lanzette, einen Energiespeicher zum Übertragen von in dem Energiespeicher gespeicherter Energie auf die Lanzette zur Ausführen einer Stechbewegung, eine Ladeeinrichtung zum Aufladen des Energiespeichers und eine Steuereinheit, wobei die Steuereinheit so ausgelegt ist, dass sie die Ladeeinrichtung im Anschluss an die Stechbewegung ansteuert, so dass der Energiespeicher im Anschluss an die Stechbewegung automatisch mit Energie für eine nächste Stechbewegung der Lanzette aufgeladen wird.

Die Erfindung bezieht sich weiterhin auf ein Verfahren zum Ausführen einer Stechbewegung mittels einer wieder verwendbaren Stechhilfe mit einer in der Stechhilfe enthaltenen Lanzette, wobei die Stechbewegung durch Übertragen von in einem Energiespeicher gespeicherter Energie auf die Lanzette erfolgt und der Energiespeicher im Anschluss an die Stechbewegung automatisch mit Energie für eine nächste Stechbewegung der Lanzette aufgeladen wird. Die Stechbewegung dient vorzugsweise zum Erzeugen einer Hautöffnung in einem Körperteil. Die Hautöffnung wird dabei vorzugsweise in einer Fingerbeere erzeugt, kann jedoch auch an einem beliebigen anderen Körperteil erzeugt werden.

Eine Stechbewegung ist in diesem Zusammenhang eine Bewegung der Lanzette, bei der die Lanzettenspitze durch eine Öffnung im Gehäuse der Stechvorrichtung bzw. im Gehäuse eines Analysesystems soweit hinausbewegt wird, dass sie ausreichend in die Haut eines an der Gehäuseöffnung anliegenden Körperteils eindringen kann. Nach der Stechbewegung wird die Lanzette vorzugsweise wieder vollständig in das Gehäuse zurückbewegt, um ein unbeabsichtigtes Stechen zu vermeiden.

Eine Lanzette kann im Zusammenhang mit der Erfindung unabhängig von anderen Komponenten, insbesondere unabhängig von einem Testelement, oder z.B. als Bestandteil eines "Disposables" in der Stechhilfe verwendet werden. In einem analytischen Hilfsmittel (Disposable) sind mehrere Funktionen bzw. Funktionselemente integriert. Insbesondere kann das analytische Hilfsmittel eine Lanzette und ein Testelement umfassen (z.B. beschrieben in US 2003/0050573 A1 oder in US 2002/0052618 A1).

Um ein möglichst schmerzarmes Einstechen zu erreichen, sollte die Lanzette eine große Beschleunigung erfahren, damit eine Einstechbewegung erfolgt, bei der die Lanzette mit hoher Geschwindigkeit in das Körperteil einsticht. Dazu ist bei dem erfindungsgemäßen Verfahren ein Energiespeicher vorgesehen, dessen gespeicherte Energie zumindest teilweise in kinetische Energie der Lanzette umgewandelt werden kann. Nach einem Einstechvorgang ist der Energiespeicher weitgehend entladen. Erfindungsgemäß wird der Energiespeicher im Anschluss an die Stechbewegung automatisch mit Energie für eine nächste Stechbewegung versorgt. Im Anschluss bedeutet dabei frühestens sofort nach dam Ausführen der Stechbewegung (bzw. sofort nach dem Erzeugen der Hautöffnung) und spätestens bevor die Stechhilfe bzw. ein die Stechhilfe umfassendes Analysegerät in einen Ruhezustand versetzt wird, der bis zu der nächsten Verwendung der Stechhilfe bzw. des Analysegeräts andauert.

Die Versorgung des Energiespeichers mit Energie für die nächste Stechbewegung erfolgt mit Hilfe einer Ladeeinrichtung, die diese Energie bereitstellt, und die durch eine Steuereinheit im Anschluss an die Stechbewegung angesteuert wird, damit sie die Energie zu diesem Zeitpunkt an den Energiespeicher abgibt. Als Steuereinheit kann z.B. ein Prozessor dienen, der in der Stechhilfe bzw. in einem die Stechhilfe umfassenden Analysegerät enthalten ist.

Bei dem erfindungsgemäßen Verfahren ergibt sich eine Vielzahl von Vorteilen, dadurch dass das Aufladen des Energiespeichers nach einem angeschlossenen Stechvorgang der Stechhilfe (gegebenenfalls nach der Durchführung einer Messung in dem Analysegerät) erfolgt, und nicht erst kurz vor der erneuten Inbetriebnahme der Stechhilfe. Die Stechhilfe ist folglich sofort einsatzbereit, wenn die Stechhilfe bzw. das Analysesystem erneut in Betrieb genommen wird. Ein Aufladen des Energiespeichers (z.B. Spannen einer Feder) muss zu diesem Zeitpunkt nicht mehr erfolgen.

Im Anschluss an eine Stechbewegung folgt hingegen üblicherweise ein längeres Zeitintervall, in dem die Stechhilfe nicht benötigt wird, so dass das Aufladen des Energiespeichers ohne Eile erfolgen kann. Wird als Ladeeinrichtung z.B. ein Elektromotor zum Aufladen des Energiespeichers eingesetzt, so kann ein relativ einfacher, schwacher Motor verwendet werden, der etwas länger arbeitet, dafür aber wenig Energie verbraucht. Somit kann die Lebensdauer der Stechhilfe (Batterien und Mechanik) erhöht werden und es können die Kosten für die Stechhilfe bzw. das Analysegerät gesenkt werden.

Ein weiterer Vorteil der vorliegenden Erfindung ist die einfache Bedienung einer Stechhilfe oder eines Analysegeräts, die/das das erfindungsgemäße Verfahren durchführt. Ein manuelles Spannen einer Feder oder ähnliches zur Vorbereitung eines Einstechvorgangs ist nicht erforderlich.

Ein weiterer vorteilhafter Aspekt der vorliegenden Erfindung ist es, dass nach dem erfindungsgemäßen Verfahren gespannte Stechhilfen während der Ruhephase vor einer erneuten Inbetriebnahme unempfindlicher gegen mechanische Einflüsse sind. Lose, ungespannte Mechaniken können sich beim Transport des Geräts bewegen und Führungen beschädigen oder ausschlagen. Nach dem erfindungsgemäßen Verfahren gespannte Systeme besitzen diesen Freiheitsgrad nicht und sind daher langlebiger.

Vorzugsweise umfasst der Energiespeicher mindestens einen Energiespeicher ausgewählt aus der Gruppe bestehend aus mindestens einer Feder, einem elektrischen Energiespeicher und einer Druckeinrichtung. Die Feder speichert Energie durch Spannen (Kompression oder Expansion). Der elektrische Energiespeicher wird mit elektrischer Energie aufgeladen. Die Druckeinrichtung enthält im aufgeladenen Zustand ein komprimiertes Gas oder Gasgemisch, das durch Entspannung seine Energie an die Lanzette zur Durchführung einer Stechbewegung abgeben kann.

Die Ladeeinrichtung umfasst vorzugsweise mindestens eine Ladeeinrichtung ausgewählt aus der Gruppe bestehend aus einem an den Energiespeicher gekoppelten Motor, einer ansteuerbaren Pumpe zur Kompression eines Gases oder Gasgemischs, einem ein Gas oder Gasgemisch enthaltenden Druckreservoir mit ansteuerbarem Ventil, einem Akku mit ansteuerbarem Schalter und einer Batterie mit ansteuerbarem Schalter. Der an den Energiespeicher gekoppelte Motor kann beispielsweise ein Elektromotor sein, der durch eine Steuereinheit ansteuerbar ist und zum Spannen einer als Energiespeicher vorgesehenen Feder für den nächsten Einstechvorgang dient. Eine als Energiespeicher vorgesehene Druckeinrichtung kann beispielsweise mit Hilfe einer ansteuerbaren Pumpe zur Kompression eines Gases oder Gasgemischs mit Energie für den nächsten Einstechvorgang versorgt werden. Es kann zu diesem Zweck aber auch ein Druckreservoir vorgesehen sein, das ein komprimiertes Gas oder Gasgemisch enthält, wobei das Druckreservoir komprimiertes Gas oder Gasgemisch an die Druckeinrichtung abgibt, sobald die Steuereinheit ein ansteuerbares Ventil öffnet. Ein elektrischer Energiespeicher kann z.B. ein Kondensator oder ein Akku sein. Ein elektrischer Energiespeicher kann durch einen als Ladeeinrichtung vorgesehenen Akku oder eine Batterie mit elektrischer Energie für den nächsten Einstechvorgang versorgt werden, wobei für den Ladevorgang eine elektrische Verbindung zwischen Energiespeicher und Ladeeinrichtung durch Ansteuern des ansteuerbaren Schalters durch die Steuereinheit hergestellt wird.

Gemäß einer bevorzugten Ausführungsform der vorliegenden Erfindung erfolgt die Stechbewegung durch Übertragen von in einer gespannten Feder gespeicherter Energie auf die Lanzette, wobei die Feder im Anschluss an die Stechbewegung erneut gespannt wird. Als Energiespeicher dient dabei die Feder, wobei das "Aufladen" des Energiespeichers durch ein Spannen der Feder erfolgt. Als Spannen wird in diesem Zusammenhang entweder das Komprimieren oder das Expandieren der Feder bezeichnet. Als Feder kann z.B. eine Spiralfeder, eine Torsionsfeder oder ein Schenkelfeder eingesetzt werden. Beim Auslösen eines Stechvorgangs wird die Feder in eine entspannte Lage geführt. Die jeweils freiwerdende Kraft wird zum Antreiben der Lanzette zur Durchführung einer Stechbewegung verwendet. Die Feder zeichnet sich durch eine schnelle Energieentnahmegeschwindigkeit aus und kann der Stechhilfe die zur Ausführung eines Stechvorgangs benötigte Energie innerhalb weniger Millisekunden zur Verfügung stellen. Die Lanzette ist vorzugsweise so über einen Kopplungsmechanismus an die Feder angekoppelt, dass die in der gespannten Feder gespeicherte Energie weitgehend als kinetische Energie auf die Lanzette übertragen werden kann. Dabei führt die Lanzette vorzugsweise eine zwangsläufig geführte Stechbewegung aus.

Gemäß einer anderen Ausführungsform der vorliegenden Erfindung wird in dem Energiespeicher gespeicherte elektrische Energie weitgehend als kinetische Energie zur Ausführung einer Stechbewegung auf die Lanzette übertragen und der Energiespeicher im Anschluss an die Stechbewegung mit elektrischer Energie aufgeladen. Ein solcher Energiespeicher, der elektrische Energie speichert, kann z.B. ein Kondensator oder Akku sein. Die elektrische Energie aus dem Energiespeicher wird zur Ausführung einer Stechbewegung vorzugsweise mittels eines Elektromagneten oder einer Tauchspule auf die Lanzette übertragen.

Gemäß einer weiteren Ausführungsform der vorliegenden Erfindung enthält der Energiespeicher ein Gas oder ein Gasgemisch, wobei der Energiespeicher im Anschluss an die Stechbewegung der Lanzette durch Kompression des Gases oder Gasgemischs und Zuführung des komprimierten Gases oder Gasgemischs mit Energie für die nächste Stechbewegung der Lanzette aufgeladen wird. Der Energiespeicher kann z.B. ein Druckluftspeicher sein, der durch Entspannen der darin enthaltenen Druckluft seine Energie als kinetische Energie auf die Lanzette überträgt.

Vorzugsweise stellt bei der vorliegenden Erfindung ein an den Energiespeicher gekoppelter Motor die Energie zum Aufladen des Energiespeichers bereit. Z.B. kann eine als Energiespeicher vorgesehene Feder durch einen Elektromotor komprimiert werden.

Zum Aufladen des Energiespeichers, insbesondere zum Spannen der Feder, kann der Motor über eine Kupplung und/oder über ein Getriebe an die Feder gekoppelt sein. Als Kupplung zwischen Motor und Energiespeicher kann z.B. eine drehmoment- oder drehwinkelgesteuerte Kupplung eingesetzt werden. Als Getriebe kann z.B. ein Kegelradgetriebe verwendet werden. Es sind jedoch alle im Stand der Technik bekannten Kupplungs- und Getriebearten denkbar, die eine Energieübertragung von dem Motor auf den Energiespeicher ermöglichen. Gemäß einer Ausführungsform der vorliegenden Erfindung stellt der Motor zusätzlich für eine weitere, von dem Energiespeicher unabhängige Systemfunktion eines Analysesystems zur Analyse einer flüssigen Probe Energie bereit. Der Motor dient dann als kombinierter Antrieb, der sowohl zum Aufladen des Energiespeichers (z.B. zum Spannen einer Feder), als auch für mindestens eine weitere Systemfunktion Energie zur Verfügung stellt. Beispielsweise kann die weitere Systemfunktion der Transport eines Testelementmagazins und/oder eines einzelnen Testelements in einem Analysesystem sein. Dabei kann der Motor nacheinander oder gleichzeitig für die verschiedenen Funktionen verwendet werden.

Vorzugsweise treibt der Motor ein Transportsystem an, das Testelemente in eine Probenaufnahmeposition und in eine Messposition transportiert. Das Testelement ist dabei vorzugsweise ein elektrochemisch oder photometrisch auszuwertender Teststreifen, der ein Testfeld umfasst, wobei das Testfeld eine Testchemie enthält, die mit einem Analyten in einer Probe reagieren kann.

Die Probenaufnahmeposition eines Testelements in einem Analysesystem ist diejenige Position, in der das Testelement zur Aufnahme einer Probe, z.B. von Blut oder interstitieller Flüssigkeit, bereit ist. Beispielsweise kann das Testelement mit einem einen Probenaufnahmeort aufweisenden Ende aus einem Schlitz im Gehäuse eines Analysesystems herausragen, so dass ein Anwender eine Blutprobe von einem Körperteil, in dem gemäß dem erfindungsgemäßen Verfahren eine Hautöffnung erzeugt worden ist, auf den Probenaufnahmeort übertragen kann.

Die Messposition eines Testelements in einem Analysesystem ist diejenige Position, in der eine Messung zur Analyse einer Probe auf dem Testelement durchgeführt wird.

Es ist eine Vielzahl von Verfahren zur Messung der Konzentration von Analyten, zum Beispiel Glukose in einer Blutprobe, bekannt. Solche Verfahren fallen üblicherweise in eine der zwei Kategorien: optische Verfahren oder elektrochemische Verfahren.

Optische Verfahren beruhen auf Farbänderungen, die sich im Verlauf der Nachweisreaktion bei Anwesenheit der zu bestimmenden Analyten zeigen. Die Detektion der auf dem Testelement auftretenden Farbänderung kann reflexionsphotometrisch erfolgen. Eine Messung in Transmission ist ebenfalls möglich, setzt jedoch zumindest teilweise transparente Teststreifen voraus.

Elektrochemische Verfahren zur Bestimmung der Konzentration eines Analyten beruhen zum Beispiel auf Amperometrie oder Coulometrie.

Zur Durchführung der elektrochemischen Analyse müssen zwischen dem Testelement und dem Analysesystem elektrische Signale übertragen werden. Daher muss ein in ein Analysesystem eingebrachtes Testelement in dem Analysesystem mit Hilfe eines elektrischen Anschlusssystems elektrisch kontaktiert werden.

Die optische oder elektrochemische Analyse erfolgt während sich das Testelement in der Messposition befindet. Die Messposition kann im Zusammenhang mit der vorliegenden Erfindung die gleiche Position des Testelements in dem Analysesystem sein, wie die Probenaufnahmeposition oder eine davon verschiedene Position.

Gemäß einer bevorzugten Ausführungsform der vorliegenden Erfindung wird das erfindungsgemäße Verfahren in einem Analysesystem durchgeführt, das ein Testelementmagazin zur Magazinierung von mindestens zwei Testelementen und eine Testelement-Entnahmeeinrichtung bzw. ein Transportsystem zur automatischen Entnahme eines Testelements aus dem Testelementmagazin und/oder zum Transport des Testelements in die Probenaufnahme- und/oder Messposition in dem Analysesystem enthält. Im Stand der Technik sind diverse Testelementmagazine bekannt.

DE 198 19 407 offenbart z.B. einen Behälter für Blutzuckermessgeräte oder andere Messgeräte, welche mit Einweg-Teststreifen arbeiten, die zur Messung einem Sensor zuführbar sind, wobei der Behälter aus zwei Teilen besteht, in dessen erstem die Teststreifen magaziniert sind und in dessen zweitem die verbrauchten Teststreifen gesammelt werden. Dabei können die Teststreifen so aneinandergereiht sein, dass sie ein Band bilden, welches ähnlich dem Band in einer Tonbandkassette gespult werden kann. Sie können stattdessen auch so angeordnet sein, dass sie eine runde Scheibe bilden, auf der sie in einem definierten Abstand zueinander im Bereich des Scheibenumfangs angeordnet sind, so dass durch Drehen der Scheibe ein neues Testfeld in die entsprechende Messposition kommt. Eine weitere Möglichkeit ist, dass die Teststreifen einen Stapel bilden, welcher durch eine durch einen Motor angetriebene Mechanik einzeln abgearbeitet wird und die Teststreifen nacheinander in die entsprechende Probenaufnahmeposition und/oder Messposition und nach Erfolgen der Messung in ein Sammelfach bringt.

In DE 198 54 316 A1 wird ein trommelförmiges Testelement-Magazin mit separaten wasserdampfdichten Kammern für Testelemente beschrieben. Jede der Kammern weist zumindest zwei gegenüberliegende, durch jeweils eine Siegelfolie verschlossene Öffnungen auf. Zur Entnahme der Testelemente erfolgt ein Herausschieben eines Testelements aus seiner Kammer mit Hilfe eines Stößels, der mittels eines Motors angetrieben wird. Der Stößel durchtrennt dabei die Siegelfolie auf der einen Seite der Kammer und drückt dann auf das Testelement, das aufgrund dieses Drucks des Stößels die Siegelfolie auf der gegenüberliegenden Seite durchtrennt, so dass das Testelement aus der Kammer herausgeschoben und in die Probenaufnahmeposition und/oder Messposition transportiert werden kann.

Gemäß einer bevorzugten Ausführungsform der vorliegenden Erfindung ist die erfindungsgemäße, wieder verwendbare Stechhilfe Bestandteil eines Analysesystems, in dem eine Analyse einer flüssigen Probe auf einem Testelement durchführbar ist.

Gemäß einer bevorzugten Ausführungsform der vorliegenden Erfindung wird eine Vielzahl von Testelementen in einem bandförmigen Testelementmagazin bereitgestellt. Die Durchführung einer Analyse einer flüssigen Probe läuft dann vorzugsweise wie folgt ab:
a) Ein Körperteil des Anwenders wird an eine Gehäuseöffnung einer Stechhilfe oder eines eine Stechhilfe enthaltenden Analysesystems angelegt, da die Lanzette der Stechhilfe bei dem erfindungsgemäßen Verfahren vorzugsweise beim Ausführen der Stechbewegung aus einer Gehäuseöffnung der Stechhilfe austritt, an die das Körperteil zum Erzeugen einer Hautöffnung anzulegen ist.
b) Auslösen des Stechvorgangs, so dass die Energie des Energiespeichers (z.B. der gespannten Feder) zumindest teilweise auf die Lanzette zum Ausführen einer Stechbewegung übertragen wird.
c) Gegebenenfalls tritt die Lanzette beim Ausführen der Stechbewegung zusätzlich durch ein Testelement hindurch, das hinter der Gehäuseöffnung z.B. als Teil eines Testelementbandes angeordnet ist. Dazu kann in dem Testelement eine entsprechend positionierte Stichöffnung vorgesehen sein, so dass der Stechbewegung der Lanzette kein Widerstand entgegensteht.
d) Die Lanzette führt eine Stechbewegung aus und die Lanzettenspitze tritt aus der Gehäuseöffnung aus, erzeugt eine Hautöffnung in dem Körperteil des Anwenders und wird wieder durch die Gehäuseöffnung und gegebenenfalls die Stichöffnung des Testelements in die Stechhilfe zurückgezogen.
e) Das Band mit Testelementen wird ggf. weiterbewegt, bis sich das zu verwendende Testelement in der Probenaufnahmeposition befindet.
f) Die flüssige Probe wird von dem Körperteil des Anwenders auf das Testelement aufgenommen.
g) Dann erfolgt die Durchführung einer Messung an der Probe (entweder in derselben Position oder das Band mit Testelementen wird in eine andere Messposition weiterbewegt).
h) Anschließend wird der Energiespeicher aufgeladen (insbesondere die Feder gespannt), so dass die Stechhilfe bzw. das Analysesystem für eine weitere Durchführung des erfindungsgemäßen Verfahrens zum Ausführen einer Stechbewegung bereit ist. Gegebenenfalls wird ferner das Band mit Testelementen weiterbewegt, so dass es ebenfalls für den nächsten Stechvorgang oder Analysevorgang bereit ist.
i) Dann folgt üblicherweise eine Ruhephase, bis die Stechhilfe/das Analysesystem erneut zum Einsatz kommt.

Die Schritte a) bis h) können in der genannten oder in einer anderen Reihenfolge nacheinander oder zumindest teilweise gleichzeitig ablaufen. Der Schritt h) läuft erfindungsgemäß immer nach dem Schritt d) und vor dem Schritt i) ab.

Das Aufladen des Energiespeichers in Schritt h) und vorzugsweise auch die Bewegung des Testelementbandes in den Schritten e), g) und h) werden durch eine Steuereinheit gesteuert. Die Steuereinheit steuert zum Aufladen des Energiespeichers eine dafür vorgesehene Ladeeinrichtung an, die dann den Energiespeicher mit Energie für den nächsten Stechvorgang versorgt.

Gemäß einer anderen Ausführungsform der vorliegenden Erfindung wird für jeden Stechvorgang gemäß dem erfindungsgemäßen Verfahren ein einzelnes Testelement oder Disposable verwendet, das der Anwender im Anschluss an einen Stechvorgang und gegebenenfalls einen Messvorgang oder im Falle eines Testelements direkt vor dem nächsten Stechvorgang manuell in das Analysesystem einlegt.

Die Erfindung bezieht sich weiterhin auf die Verwendung einer Stechhilfe zum Ausführen einer Stechbewegung (insbesondere zum Erzeugen einer Hautöffnung in einem Körperteil) mittels einer wieder verwendbaren Stechhilfe mit einer in der Stechhilfe enthaltenen Lanzette, wobei die Stechbewegung durch Übertragen von in einem Energiespeicher gespeicherter Energie auf die Lanzette erfolgt, wobei der Energiespeicher im Anschluss an die Stechbewegung automatisch mit Energie für eine nächste Stechbewegung der Lanzette aufgeladen wird.

Anhand der Zeichnung wird die Erfindung nachstehend näher erläutert.

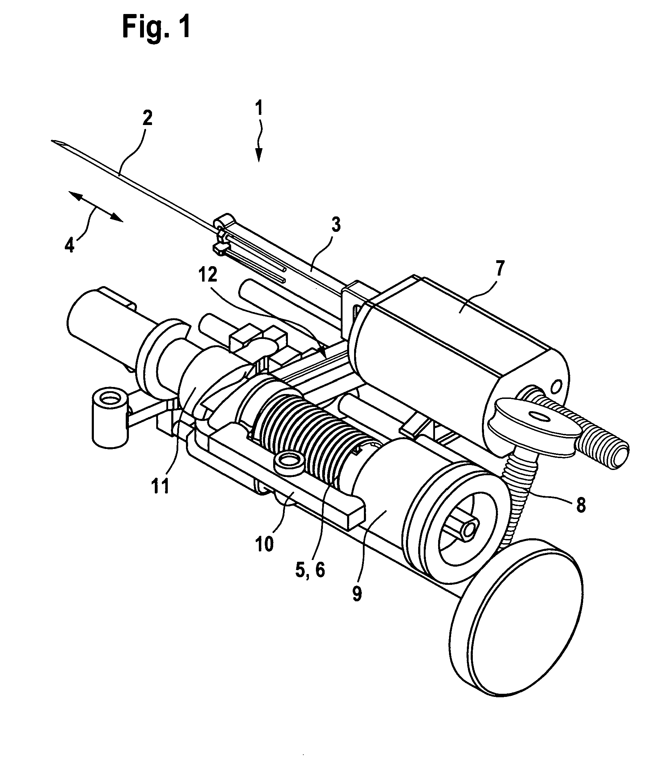

Es zeigt
- Figur 1: eine Stechhilfe zum Ausführen einer Stechbewegung gemäß dem erfindungsgemäßen Verfahren.

Die Stechhilfe 1 kann Bestandteil eines (nicht dargestellten) Analysesystems sein oder einzeln vorliegen. Sie umfasst eine Lanzette 2, die durch einen Lanzettenkörper 3 gehalten wird. Die Lanzette 2 und der Lanzettenkörper 3 sind gemeinsam linear in Längsrichtung 4 bewegbar, um eine Stechbewegung durchzuführen. Als Energiespeicher 5 dient in der in Figur 1 dargestellten Ausführungsform eine Feder 6, die die Energie für die Stechbewegung bereitstellen kann.

Diese Energie wird in dem Energiespeicher 5 durch Spannen der Feder 6 gespeichert. Zum Spannen der Feder 6 ist als Ladeeinrichtung ein Motor 7 vorgesehen, der über ein Getriebe 8 den Spannrotor 9 drehen kann. Der Motor 7 ist durch eine (nicht dargestellte) Steuereinheit ansteuerbar. Durch die Drehbewegung des Spannrotors 9 wird die Feder 6 gespannt, bis sie ausreichend Energie für die Stechbewegung bespeichert hat.

Zum Auslösen der Stechbewegung wird der Spannrotor 9 durch den Motor 7 um einen kleinen vorbestimmten Winkel in Spannrichtung weitergedreht, bis ein (nicht dargestellter) Nocken an dem Spannrotor 9 den Kipphebel 10 betätigt, der einen (nicht dargestellten) Auslöser dieses Antriebsrotors 11 drückt. Dann wird die Energie der Feder 6 auf den Antriebsrotor 11 übertragen, wodurch der Lanzettenkörper 3 und die Lanzette 2 mittels einer Steuerkurve und eines Hebels 12 zur Durchführung einer Stechbewegung angetrieben werden. Dabei wird die im Energiespeicher 5 (Feder 6) gespeicherte Energie zumindest teilweise in die kinetische Energie der Lanzette 2 bei dem Stechvorgang umgesetzt. Erfindungsgemäß wird der Energiespeicher 5 im Anschluss an die Stechbewegung automatisch für die nächste Stechbewegung der Lanzette 2 aufgeladen. Dies erfolgt durch ein Ansteuern des Motors 7 durch die (nicht dargestellte) Steuereinheit und ein erneutes Spannen der Feder 6 mit Hilfe des Motors 7 und des Getriebes 8.

### Bezugszeichenliste

- 1: Stechhilfe
- 2: Lanzette
- 3: Lanzettenkörper
- 4: Längsrichtung
- 5: Energiespeicher
- 6: Feder
- 7: Motor
- 8: Getriebe
- 9: Spannrotor
- 10: Kipphebel
- 11: Antriebsrotor
- 12: Hebel

## Patentansprüche

1. Wieder verwendbare Stechhilfe (1), enthaltend eine Lanzette (2), einen Energiespeicher (5) zum Übertragen von in dem Energiespeicher (5) gespeicherter Energie auf die Lanzette (2) zur Ausführen einer Stechbewegung, eine Ladeeinrichtung zum Aufladen des Energiespeichers (5) und eine Steuereinheit, **dadurch gekennzeichnet, dass** die Steuereinheit so ausgelegt ist, dass die Ladeeinrichtung im Anschluss an die Stechbewegung durch die Steuereinheit angesteuert wird, so dass der Energiespeicher (5) im Anschluss an die Stechbewegung automatisch mit Energie für eine nächste Stechbewegung der Lanzette (2) aufgeladen wird.

2. Stechhilfe gemäß Anspruch 1, **dadurch gekennzeichnet, dass** der Energiespeicher (5) mindestens einen Energiespeicher (5) ausgewählt aus der Gruppe bestehend aus einer Feder (6), einem elektrischen Energiespeicher und einer Druckeinrichtung und dass die Ladeeinrichtung mindestens eine Ladeeinrichtung ausgewählt aus der Gruppe bestehend aus einem an den Energiespeicher gekoppelten Motor (7), einer ansteuerbaren Pumpe zur Kompression eines Gases oder Gasgemischs, einem ein Gas oder Gasgemisch enthaltenden Druckreservoir mit ansteuerbarem Ventil, einem Akku mit ansteuerbarem Schalter und einer Batterie mit ansteuerbarem Schalter umfasst.

3. Verfahren zum Ausführen einer Stechbewegung mittels einer wieder verwendbaren Stechhilfe mit einer in der Stechhilfe enthaltenen Lanzette, wobei die Stechbewegung durch Übertragen von in einem Energiespeicher gespeicherter Energie auf die Lanzette erfolgt, **dadurch gekennzeichnet, dass** eine Steuereinheit eine Ladeeinrichtung so ansteuert, dass die Ladeeinrichtung den Energiespeicher im Anschluss an die Stechbewegung automatisch mit Energie für eine nächste Stechbewegung der Lanzette auflädt.

4. Verfahren gemäß Anspruch 3, **dadurch gekennzeichnet, dass** die Stechbewegung durch Übertragen von in einer gespannten Feder gespeicherter Energie auf die Lanzette erfolgt, wobei die Feder im Anschluss an die Stechbewegung erneut gespannt wird.

5. Verfahren gemäß Anspruch 3, **dadurch gekennzeichnet, dass** in dem Energiespeicher gespeicherte elektrische Energie zumindest teilweise als kinetische Energie zur Ausführung einer Stechbewegung auf die Lanzette übertragen wird und der Energiespeicher im Anschluss an die Stechbewegung mit elektrischer Energie aufgeladen wird.

6. Verfahren gemäß Anspruch 3, **dadurch gekennzeichnet, dass** der Energiespeicher ein Gas oder Gasgemisch enthält, wobei der Energiespeicher im Anschluss an die Stechbewegung der Lanzette durch Kompression des Gases oder Gasgemischs mit Energie für die nächste Stechbewegung der Lanzette aufgeladen wird.

7. Verfahren gemäß einem der Ansprüche 3 bis 6, **dadurch gekennzeichnet, dass** als Ladeeinrichtung ein an den Energiespeicher gekoppelter Motor die Energie zum Aufladen des Energiespeichers bereitstellt.

8. Verfahren gemäß Anspruch 7, **dadurch gekennzeichnet, dass** der Motor zusätzlich für eine weitere von dem Energiespeicher unabhängige Systemfunktion eines Analysesystems zur Analyse einer flüssigen Probe Energie bereitstellt.

9. Verfahren gemäß einem der Ansprüche 7 oder 8, **dadurch gekennzeichnet, dass** der Motor ein Transportsystem antreibt, das Testelemente in eine Probenaufnahmeposition und in eine Messposition transportiert.

10. Verfahren gemäß einem der Ansprüche 3 bis 9, **dadurch gekennzeichnet, dass** die Lanzette beim Ausführen der Stechbewegung aus einer Gehäuseöffnung der Stechhilfe austritt, an die ein Körperteil zum Erzeugen einer Hautöffnung anzulegen ist.

11. Verfahren gemäß einem der Ansprüche 3 bis 10, **dadurch gekennzeichnet, dass** die Lanzette beim Ausführen der Stechbewegung zusätzlich durch ein Testelement hindurchtritt.

12. Verwendung einer Stechhilfe zum Ausführen einer Stechbewegung mit einer in der Stechhilfe enthaltenen Lanzette, wobei die Stechbewegung durch Übertragen von in einem Energiespeicher gespeicherter Energie auf die Lanzette erfolgt, **dadurch gekennzeichnet, dass** der Energiespeicher im Anschluss an die Stechbewegung automatisch mit Energie für eine nächste Stechbewegung der Lanzette aufgeladen wird.
